# EUROPEAN PATENT APPLICATION

(11) **EP 4 810 605 A1**
(43) Date of publication of application: **23.09.2026**
(21) Application number: 24891481.4
(22) Date of filing: 14.11.2024
(51) Int. Cl.: C12N 9/50, A23C 20/02, C12N 9/10, C12N 9/24

(54) **ENZYME PREPARATION FOR CHEESE ANALOG**

(30) Priority: 14.11.2023 JP 2023194015
(71) Applicant: AJINOMOTO CO., INC., Chuo-ku Tokyo 104-8315 (JP)
(72) Inventor: HAMAMOTO, Dai, Kawasaki-shi, Kanagawa 210-8681 (JP); HORI, Tetsuo, Kawasaki-shi, Kanagawa 210-8681 (JP); SOGA FUJIMURA, Yuko, Kawasaki-shi, Kanagawa 210-8681 (JP); YAMAMOTO, Yukio, Kawasaki-shi, Kanagawa 210-8681 (JP)
(74) Representative: Strehl & Partner mbB
(86) International application number: PCT/JP2024/040527
(87) International publication number: WO 2025/105445

(57) **Abstract**

The present invention provides an enzyme preparation for cheese analogs, containing (A) a protease, (B) a carbohydrate-modifying enzyme, and (C) a transglutaminase, which improves both the meltability and stretchability when heated and the shreddability when cooled of cheese analogs, as well as a method for producing cheese analogs improved in the above-mentioned properties.

## Description

### [Technical Field]

The present invention relates to an enzyme preparation for cheese analogs that improves both the properties during heating and shreddability during cooling, a method for producing a cheese analog possessing the aforementioned properties, and a method for increasing the hardness of a cheese analog possessing properties during heating.

### [Background Art]

In recent years, due to growing awareness of animal welfare and health consciousness, the market for dairy-free cheeses using no dairy ingredients (plant-based protein) and cheese analogs has been expanding. As a dairy-free cheese substitute, a production method is known that uses plant-based milk that does not contain dairy components and employs enzymes such as protease, lipase, and transglutaminase, as well as microorganisms (Patent Literature 1). On the other hand, cheese analogs, also called analog cheese, imitation cheese, or the like, are foods in which starch is added, and some or all of the fat and protein of cheese are replaced with plant-derived components, and which can be produced without fermentation or maturation steps and are processed to have the same appearance and texture as cheese. However, in addition to the appearance and texture at room temperature, good meltability and/or stretchability when heated are also desired from the viewpoints of cooking suitability, texture, and the like. In this connection, it is already known to react proteases and α-glucosidases with cheese analogs to impart meltability and/or stretchability when heated, similar to those of cheese (Patent Literature 2).

It is also known to react cheese analogs with transglutaminase in order to impart a smooth texture (Patent Literature 3).

Furthermore, the demand for cheese analogs such as shredded cheese and sliced cheese, which are widely used in both household and commercial fields, such as as toppings and ingredients in pizzas, salads, and snack products, is also increasing.

### [Citation List]

### [Patent Literature]

[Patent Literature 1]
   JP 2016-502868 A
[Patent Literature 2]
   WO2023/033188
[Patent Literature 3]
   WO2023/033187

### [Summary of Invention]

### [Technical Problem]

The present invention has found a problem that, when a cheese analog with improved meltability and/or stretchability when heated is produced by reacting a carbohydrate-modifying enzyme such as α-glucosidase (also abbreviated as AG) and a protease with a cheese analog containing starch and protein, the hardness of the cheese analog decreases during cooling (during production), which in turn decreases so-called shreddability in a finely-cutting step, thus resulting in a decline in production suitability. Therefore, the present invention aims to provide a technique that improves both the properties when heated and the shreddability when cooled.

### [Solution to Problem]

The present inventors intensively studied to solve the above-mentioned problems and found that a cheese analog that maintains properties during heating while improving hardness during cooling and improving shreddability can be produced by reacting a cheese analog with a protease and a carbohydrate-modifying enzyme such as AG or glucoamylase (also abbreviated as GA), and further with transglutaminase (also abbreviated as TG). Based on the above-mentioned finding, the present inventors have further studied intensively and completed the present invention.

That is, the present invention provides the following.
[1] An enzyme preparation for a cheese analog, comprising (A) a protease, (B) a carbohydrate-modifying enzyme, and (C) a transglutaminase.
[2] The preparation of [1], wherein (B) is α-glucosidase, glucoamylase, or a mixture thereof.
   [2-1] The preparation of [1], wherein (B) is glucoamylase.
[3] The preparation of [1], [2], or [2-1], wherein the cheese analog comprises a protein, starch, and fat or oil.
[4] The preparation of any of [1] to [3], wherein the cheese analog is a finely-cut cheese analog.
[5] The preparation of [4], wherein the finely-cut cheese analog is a shredded cheese analog.
[6] The preparation of any of [1] to [5], comprising 100 to 1000000 U of (A), 0.1 to 100000 U of (B), and 0.1 to 200 U of (C), per 1 g of the enzyme preparation.
   [6-1] The preparation of [6], comprising 0.1 to 100 U of α-glucosidase as (B) per 1 g of the enzyme preparation.
   [6-2] The preparation of [6], comprising 0.1 to 100000 U of glucoamylase as (B) per 1 g of the enzyme preparation.
[7] The preparation of any of [6] to [6-2], which is added in an amount of 0.1 to 50% by weight relative to the total weight of the cheese analog.
[8] A method for producing a cheese analog, comprising (1) a step of reacting a mixture comprising a protein, starch, and fat or oil with (A) protease, (B) a carbohydrate-modifying enzyme, and (C) transglutaminase.
[9] The production method of [8], wherein (B) is α-glucosidase, glucoamylase, or a mixture thereof.
   [9-1] The production method of [8], wherein (B) is glucoamylase.
[10] The production method of [8], [9], or [9-1], wherein the protein is a plant-derived protein.
[11] The production method of any of [8] to [10], further comprising
   (2) a step of heating the mixture obtained in step (1),
   (3) a step of cooling the heated mixture, and
   (4) a step of cutting the cooled mixture.
[12] The production method of [11], wherein the cheese analog is a finely-cut cheese analog.
[13] The production method of [12], wherein the finely-cut cheese analog is a shredded cheese analog.
[14] The production method of any of [8] to [13], wherein step (1) is a step of reacting 0.001 to 100000 U of (A), 0.0001 to 10000 U of (B), and 0.0001 to 1000 U of (C), per 1 g of the mixture.
   [14-1] The production method of any of [8] to [13], wherein step (1) is a step of reacting 0.001 to 100000 U of (A), 0.0001 to 100000 U of (B), and 0.0001 to 1000 U of (C), per 1 g of the mixture.
   [14-2] The production method of [14-1], wherein α-glucosidase as (B) is reacted at 0.0001 to 10000 U per 1 g of the mixture in step (1).
   [14-3] The production method of [14-1], wherein glucoamylase as (B) is reacted at 0.001 to 100000 U per 1 g of the mixture in step (1).
[15] A method for increasing the hardness of a cheese analog, comprising a step of reacting a mixture comprising a protein, starch, and fat or oil with (A) a protease, (B) a carbohydrate modifying enzyme, and (C) a transglutaminase.

### [Advantageous Effects of Invention]

According to the present invention, cheese analogs of shredded type and sliced type can be produced without waste because the cheese exhibits appropriate meltability and stretchability when heated, but maintains appropriate hardness when cut finely.

### [Brief Description of Drawings]

[Fig. 1]

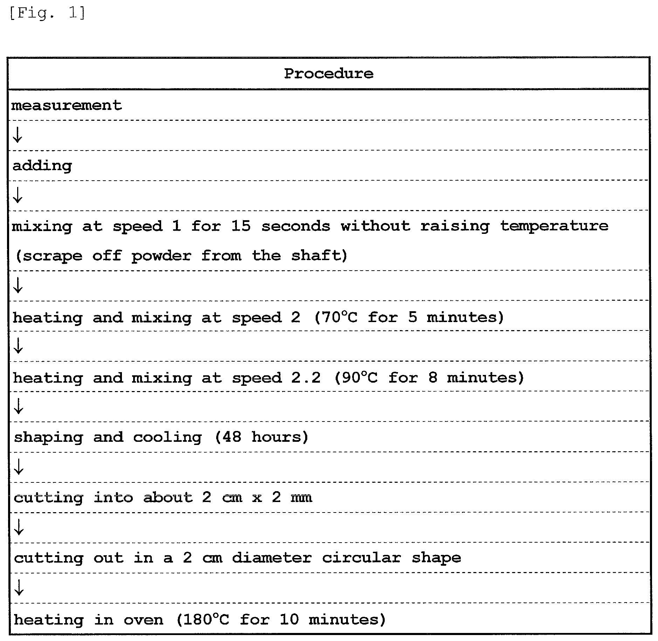

   Fig. 1 shows the production flowchart for cheese analogs.
[Fig. 2]

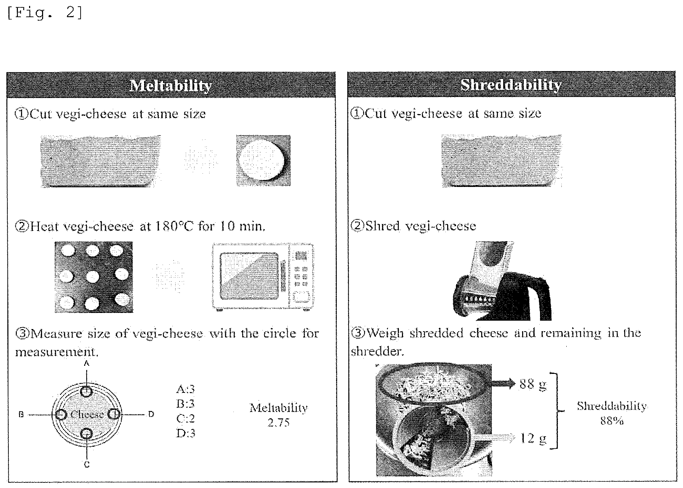

   Fig. 2 shows the calculation method for the meltability and shreddability of cheese analogs.
[Fig. 3]

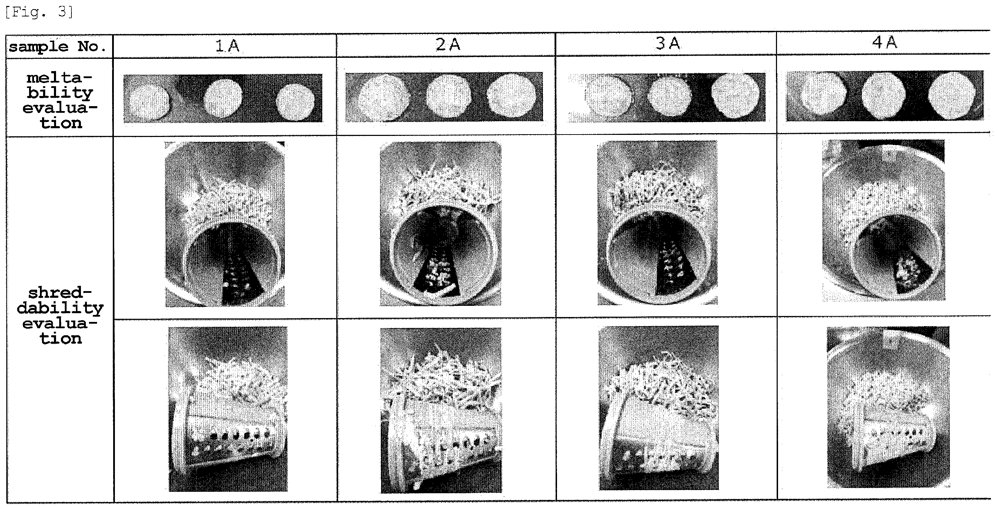

   Fig. 3 shows photographs observing the meltability and shreddability of cheese analogs when heated in Experimental Example 1.
[Fig. 4]

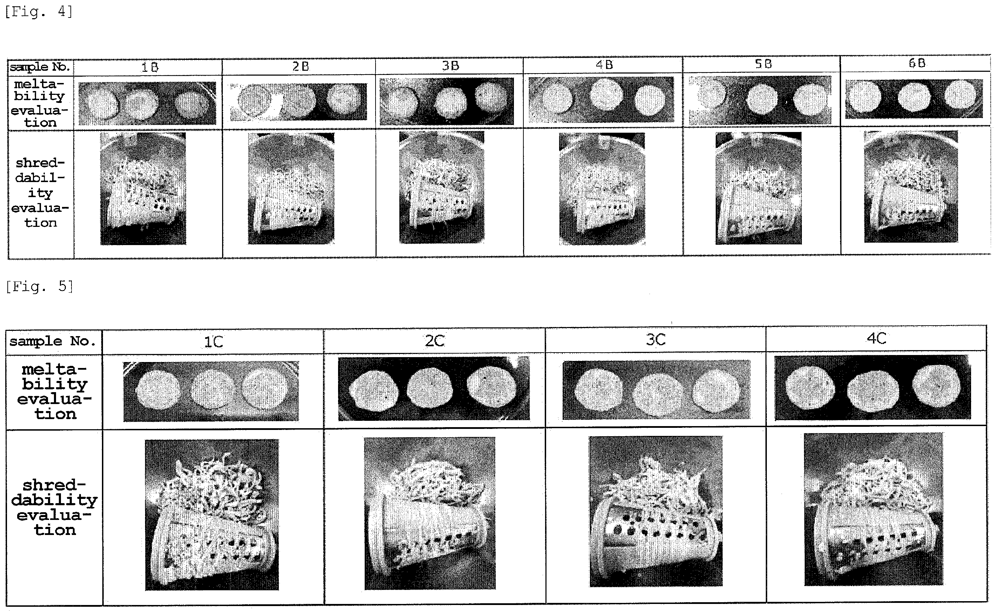

   Fig. 4 shows photographs observing the meltability and shreddability of cheese analogs when heated in Experimental Example 2.
[Fig. 5]
   Fig. 5 shows photographs observing the meltability and shreddability of cheese analogs when heated in Experimental Example 3.
[Fig. 6]

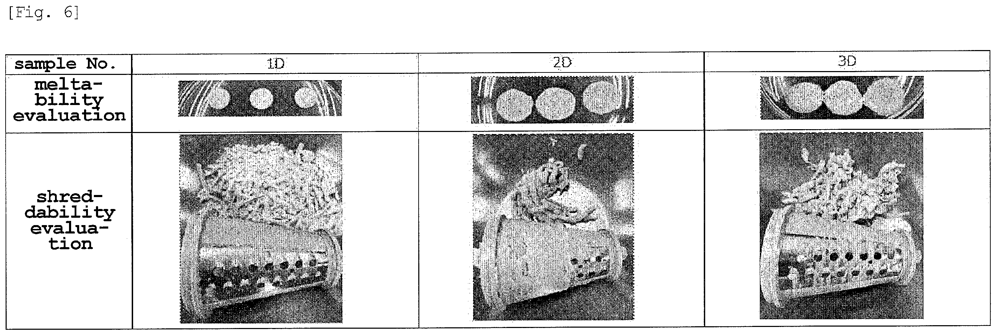

   Fig. 6 shows photographs observing the meltability and shreddability of cheese analogs when heated in Experimental Example 4.

### [Description of Embodiments]

### 1. Enzyme preparation

The present invention relates to an enzyme preparation for cheese analog, containing (A) protease, (B) carbohydrate-modifying enzyme, and (C) transglutaminase (also abbreviated as the preparation of the present invention).

### (A) Protease

The protease used in the present invention is an enzyme that catalyzes the hydrolysis of peptide bonds in proteins. In the present invention, proteases having any substrate specificity and any reaction property can be used as long as they have such activity and can degrade proteins. Furthermore, the origin thereof is not particularly limited, and one derived from any source, such as plants (e.g., papaya), mammals, fish, or microorganisms (e.g., genus Aspergillus, genus Bacillus, or genus Rhizopus) can be used, and recombinant enzymes may also be used.

In the present invention, the active unit of endoprotease (1 U) is defined as the amount of enzyme that produces an increase in a Folin reagent color-developing substance corresponding to 1 µg of tyrosine per minute, using casein as a substrate.

In the present invention, the active unit (1 U) of exoprotease is defined as the activity that generates 1 µmol of p-nitroaniline per minute, using L-leucyl-p-nitroanilide as a substrate.

In the present invention, the protease includes endo/exoprotease, endoprotease, exoprotease, and combinations of these.

In the preparation of the present invention, protease is preferably selected from the group consisting of (1) endo/exoprotease, (2) endoprotease and exoprotease in combination, (3) exoprotease, and (4) endoprotease; more preferably selected from the group consisting of (1) endo/exoprotease, (2) endoprotease and exoprotease in combination, and (4) endoprotease; further preferably selected from the group consisting of (1) endo/exoprotease and (2) endoprotease and exoprotease in combination.

The endo/exoprotease used in the present invention is an enzyme that hydrolyzes peptide bonds within proteins and at the ends of proteins, and breaks them into several peptides or amino acids.

The endo/exoprotease used in the present invention may be a commercially available product and, for example, ProteAX (manufactured by Amano Enzyme Inc.; derived from Aspergillus oryzae), peptidase R (manufactured by Amano Enzyme Inc.; derived from Rhizopus oryzae), Denazyme AP (manufactured by Nagase ChemteX Corporation; derived from Aspergillus oryzae), food-grade refined papain (manufactured by Nagase ChemteX Corporation; derived from papaya latex) can be mentioned.

The endoprotease used in the present invention is an enzyme that hydrolyzes peptide bonds within proteins, producing several peptides.

The endoproteases used in the present invention may be commercially available products, such as Protin SD-NY10 (manufactured by Amano Enzyme Inc.; derived from Bacillus amyloliquefaciens), Protin SD-AY10 (manufactured by Amano Enzyme Inc.; derived from Bacillus licheniformis), Denapsin 2P (manufactured by Nagase ChemteX Corporation; derived from Aspergillus niger), and Bioplase SP-20FG (manufactured by Nagase ChemteX Corporation; derived from Bacillus licheniformis).

The exoprotease used in the present invention is an enzyme that hydrolyzes peptide bonds at the amino terminus or carboxyl terminus of a protein, releasing amino acids.

The exoprotease used in the present invention may be a commercially available product. The exoprotease used in the present invention may be, for example, aminopeptidase (purified product). For example, Denazyme LEP 10P (manufactured by Nagase ChemteX Corporation) can be mentioned.

### (B) Carbohydrate-modifying enzyme

The carbohydrate-modifying enzymes used in the present invention are classified into enzymes having glycolytic activity, enzymes having glycosyltransferase activity, and enzymes that oxidize sugars. The carbohydrate-modifying enzyme used in the present invention is not particularly limited as long as it does not inhibit the effect of the present invention. Among those, enzymes having glycolytic activity (also to be referred to as carbohydrate-degrading enzymes), which hydrolyze the glycosidic bonds of carbohydrates, are preferred.

In the present invention, carbohydrate-modifying enzymes include carbohydrate-degrading enzymes such as α-amylase, β-amylase, invertase, maltotriohydrolase, pullulanase, amyloglucosidase, α-glucosidase, β-glucosidase, isoamylase, glucoamylase, pectinase, cellulase, and hemicellulase. Among these, α-glucosidase (EC3.2.1.20) and glucoamylase, which are exo-type enzymes that hydrolyze α-1,4 glycosidic bonds at the glucose level to produce α-glucose, are preferred.

The origin of the carbohydrate-modifying enzyme is not particularly limited and may be from plants, animals, or microorganisms. Specific examples include enzymes derived from filamentous fungi, preferably the genus Aspergillus, more preferably Aspergillus niger. Recombinant enzymes created using genetic engineering techniques may also be used.

These carbohydrate-modifying enzymes may be used alone or in combination of two or more kinds thereof. Those produced by conventional methods may be used or commercially available products may be used.

1U (unit) of α-glucosidase is defined as the amount of enzyme that produces 1 µg of glucose in 60 min under conditions of pH 5.0, 40°C, using α-methyl-D-glucoside as a substrate (Reference: Japan Food Additives Association, 4th Edition, Voluntary Standards for Existing Additives, "Method for Measuring Transglucosidase Activity").

1U (unit) of glucoamylase is defined as the activity required to generate reducing power corresponding to 10 mg of glucose from soluble starch in 30 min under conditions of pH 5.0, 40°C.

### (C) Transglutaminase

Transglutaminase (protein-glutamine-γ-glutamyltransferase) is a transferase that catalyzes a reaction in which the amino group of a glutamine residue in a protein condenses with a primary amine, transferring substituents on the amine to the glutamine residue, to produce ammonia. Generally, the amino group of a lysine residue in a protein is used as the primary amine, and it acts as a cross-linking enzyme.

As transglutaminase, calcium-independent transglutaminases obtained from microorganisms are preferably used.

Examples of calcium-independent transglutaminases derived from microorganisms include transglutaminases produced by actinomycetes belonging to the genus Streptomyces. They can be obtained according to the methods described in JP-2572716 B, and commercially available products such as "Activa TG-K" and "Activa TG-S" provided by Ajinomoto Co., Inc. can also be used.

The enzyme activity of transglutaminase can be measured and calculated, for example, by the hydroxamate method. Specifically, a reaction is performed using benzyloxycarbonyl-L-glutaminylglycine and hydroxylamine as substrates, and an iron complex of the hydroxamic acid produced in the reaction is formed in the presence of trichloroacetic acid. Then, the absorbance at 525 nm is measured, and the amount of hydroxamic acid produced is determined from a calibration curve, whereby the enzyme activity can be calculated. In the present specification, the amount of enzyme that produces 1 µmol of hydroxamic acid per min at 37°C, pH=6.0 is defined as 1 U (see JP 64-027471 A).

The cheese analog of the present invention is a cheese-like food product produced by reacting (A) to (C) with a mixture containing protein, carbohydrates such as starch, and fats and oils, and is a cheese substitute. Cheese analogs include those that use milk-derived raw materials such as milk protein and cheese for flavor, and plant-based (plant-derived) cheese analogs that do not use animal protein. The cheese analog of the present invention is preferably a plant-based cheese analog.

The "protein" used in the present invention is a protein generally used in food applications, and includes non-animal-derived proteins such as plant-derived proteins, microbial-derived proteins, and fungi-derived proteins.

Examples of the protein in the present invention include plant-derived proteins such as almond protein, soy protein, pea protein, chickpea protein, broad bean protein, lentil protein, oat protein, chia seed protein, rapeseed protein, floating grass protein, and the like; proteins derived from microorganisms; proteins derived from fungi, and the like. Among these, plant-derived proteins are preferred, almond protein, soy protein, pea protein, broad bean protein, and lentil protein are preferred, and pea protein, broad bean protein, and lentil protein are more preferred. One kind of protein may be used, or two or more kinds may be used in combination.

In the cheese analog of the present invention, the content of protein is generally 0.1% by weight or more, preferably 0.2% by weight or more, more preferably 0.5% by weight or more, further preferably 1% by weight or more, further more preferably 2% by weight or more, 3% by weight or more, 4% by weight or more, or 5% by weight or more, particularly preferably 8% by weight or more or 12% by weight or more, relative to the total weight of the cheese analog, and the upper limit is generally 50% by weight or less, preferably 40% by weight or less, more preferably 30% by weight or less, further preferably 20% by weight or less, particularly preferably 16% by weight or less.

Specifically, in the cheese analog of the present invention, the content of protein is generally 0.1 to 50% by weight, preferably 0.2 to 40% by weight, more preferably 0.5 to 30% by weight, further preferably 1 to 20% by weight, further more preferably 2 to 20% by weight, 3 to 20% by weight, 4 to 20% by weight, or 5 to 20% by weight, particularly preferably 8 to 16% by weight or 12 to 16% by weight, relative to the total weight of the cheese analog.

In the cheese analog of the present invention, if the content of protein is less than 0.1% by weight relative to the cheese analog, the cheese analog tends to have an insufficient nutritional value, and oil separation is more likely to occur during production. If the content exceeds 50% by weight, the cheese analog tends to have a powdery texture that does not taste good, and also tends to gel easily during production.

The "starch" used in the present invention refers to plant-derived raw or processed starch generally used in food products.

Examples of the starch in the present invention include rice starch, sago starch, tapioca starch, waxy corn starch, regular corn starch, potato starch, wheat starch, dry heat-treated starches of these plant-derived starches, and chemically treated starches of these plant-derived starches, such as hydroxypropylated phosphate cross-linked starch, acetylated adipic acid cross-linked starch, acetylated phosphate cross-linked starch, acetylated oxidized starch, sodium octenyl succinate starch, starch acetate, oxidized starch, hydroxypropyl starch, phosphate monoesterified phosphate cross-linked starch, phosphorylated starch, and phosphate cross-linked starch. Waxy corn starch and tapioca starch are preferred. One kind of starch may be used or two or more kinds thereof may be used in combination.

In the cheese analog of the present invention, the content of starch is generally 5% by weight or more, preferably 7.5% by weight or more, more preferably 10% by weight or more, further preferably 12.5% by weight or more, particularly preferably 18% by weight or more, relative to the total weight of the cheese analog, and the upper limit is generally 70% by weight or less, preferably 60% by weight or less, more preferably 50% by weight or less, further preferably 40% by weight or less, particularly preferably 22% by weight or less.

Specifically, in the cheese analog of the present invention, the content of starch is generally 5 to 70% by weight, preferably 7.5 to 60% by weight, more preferably 10 to 50% by weight, further preferably 12.5 to 40% by weight, particularly preferably 18 to 22% by weight, relative to the total weight of the cheese analog.

In the cheese analog of the present invention, the effect of imparting a smooth texture and shape retention similar to milk cheese can be obtained by setting the content of starch to fall within the above-mentioned range.

In the cheese analog of the present invention, if the content of starch is less than 5% by weight relative to the total weight of the cheese analog, the cheese analog tends to show insufficient shape retention, becomes excessively soft, and does not taste good. If the content exceeds 70% by weight, the cheese analog tends to have a powdery texture that does not taste good, and the viscosity of the mixture during production tends to increase excessively, making mixing difficult.

The "fat or oil" used in the present invention refers to fat or oil generally used in food products.

Examples of the fat or oil in the present invention include plant-derived fats and oils, vegetable oils such as coconut oil, palm oil, rapeseed oil, soybean oil, corn oil, safflower oil, and cocoa oil; and animal-derived fat or oil such as beef tallow, pork tallow, and chicken tallow. Plant-derived fat or oil are preferred, and coconut oil is more preferred. One kind of fat or oil may be used or two or more kinds thereof may be used in combination.

In the cheese analog of the present invention, the content of fat or oil is generally 2.5% by weight or more, preferably 5% by weight or more, more preferably 7.5% by weight or more, further preferably 10% by weight or more, particularly preferably 18% by weight or more, relative to the total weight of the cheese analog, and the upper limit is generally 70% by weight or less, preferably 60% by weight or less, more preferably 50% by weight or less, further preferably 40% by weight or less, particularly preferably 22% by weight or less.

More specifically, in the cheese analog of the present invention, the content of fat or oil is generally 2.5 to 70% by weight, preferably 5 to 60% by weight, more preferably 7.5 to 50% by weight, further preferably 10 to 40% by weight, particularly preferably 18 to 22% by weight, relative to the total weight of the cheese analog.

In the cheese analog of the present invention, if the content of fat or oil is less than 2.5% by weight relative to the total weight of the cheese analog, the cheese analog tends to have a powdery texture that does not taste good, and the melting property after heating tends to be insufficient. If the content exceeds 70% by weight, the cheese analog tends to show insufficient shape retention, becomes excessively soft, and does not taste good. In addition, oil separation is more likely to occur during production.

In the cheese analog of the present invention, the weight ratio of starch and protein (starch: protein) is generally 1:0.001 to 100, preferably 1:0.005 to 80, more preferably 1:0.01 to 50, further preferably 1:0.025 to 40.

In the cheese analog of the present invention, the weight ratio of starch and protein and fat or oil (starch:protein:fat or oil) is generally 1:0.001 to 100:0.001 to 100, preferably 1:0.005 to 80:0.005 to 80, more preferably 1:0.01 to 50:0.01 to 50, further preferably 1:0.025 to 40:0.025 to 40.

The cheese analogs in the present invention may contain additives generally used in the food field and other than the above-mentioned components.

Examples of additives include flavors (e.g., cheddar flavor (powder, liquid), parmesan flavor (powder, liquid), camembert flavoring (powder, liquid), cream cheese flavor (powder, liquid)), seasonings (e.g., salt, yeast extract, lactic acid), colorants, excipients (dextrin, lactose), various amino acids, thickening polysaccharides (e.g., gum arabic, xanthan gum, tamarind seed gum, guar gum, locust bean gum, carrageenan, agar), proteases, carbohydrate modifying enzymes, and enzymes other than TG.

The amount of the additive to be used is, for example, 1 to 30% by weight relative to the cheese analog.

The cheese analog of the present invention contains the above-mentioned components, and a block cheese analog produced by the action of the above-mentioned enzyme and through the below-mentioned steps may be provided as is, but the cheese analog is preferably in the form of sliced or finely-cut (shredded) cheese. Such cheese is also called cut cheese, sheared cheese, shredded cheese, or diced cheese, and the enzyme preparation of the present invention is preferably applied to finely-cut cheese such as sliced cheese or shredded cheese.

The enzyme preparation for cheese analogs of the present invention improves the quality (e.g., texture and appearance) or production suitability of cheese analogs. Texture refers to pleasant sensations, such as chewiness and mouthfeel, when food, improved in meltability and/or stretchability when heated, is placed in the mouth. Appearance refers to an appetizing form suitable for the dish. Production suitability refers to whether or not it is suitable for production at the desired quality level. When cutting cheese analogs, it means that there is no sticking to the finely-cutting machine, the binding of finely-cut cheese pieces is suppressed, and the like.

In the present invention, "heated" in "meltability and/or stretchability when heated" refers to heating to melt and/or stretch the cheese analog, for example, during cooking or food processing using the cheese analog as a food ingredient. The heating temperature is, for example, 70 to 200°C, and the heating time is, for example, 1 to 20 min. "When heated" refers to immediately after heating is completed (for example, within 20 min).

In the present invention, "meltability" refers to the property of the cheese analog to liquefy, melt, and spread.

In the present invention, "stretchability" refers to the property of the cheese analog to stretch like a string.

In the present invention, "improvement in meltability when heated" means that the meltability of the cheese analog produced with the addition of the enzyme of the present invention is improved when heated, compared to the meltability of the cheese analog produced without the addition of the enzyme when heated.

In the present invention, "improvement in stretchability when heated" means that the stretchability of the cheese analog produced with the addition of the enzyme of the present invention is improved when heated, compared to the stretchability of the cheese analog produced without the addition of the enzyme when heated.

In the present specification, the effect of improving "meltability and/or stretchability when heated" can be evaluated, for example, by the method described in the below-mentioned Example or a method analogous thereto.

The form of the enzyme preparation of the present invention is not particularly limited as long as it includes (A) to (C). For example, the preparation of the present invention may include (A) to (C) together, or it may be in the form of a kit that is prepared separately and combined before use.

When (A) to (C) are included together, the activity of each enzyme contained per gram of the enzyme preparation of the present invention is as follows:
(A): endo- or endo/exo-protease: generally 100 to 1000000U, preferably 2000 to 100000U, more preferably 10000 to 50000U,
(B): carbohydrate-modifying enzyme: generally 0.1 to 100000U, preferably 1 to 20000U, more preferably 5 to 5000U,
   in the case of α-glucosidase: generally 0.1 to 100U, preferably 1 to 50U, more preferably 5 to 40U,
   in the case of glucoamylase: generally 0.1 to 100000U,
   preferably 1 to 20000U, more preferably 10 to 5000U, and
(C): generally 0.1 to 200U, preferably 1 to 100U, more preferably 10 to 50U.

More specifically, when (A) to (C) are included together in the enzyme preparation of the present invention, the ratio of each enzyme to the total amount of enzymes contained in the enzyme preparation of the present invention is as follows:
(A): endo- or endo/exo-protease: generally 1 to 40 parts by weight, preferably 5 to 20 parts by weight,
(B): in the case of α-glucosidase: generally 1 to 40 parts by weight, preferably 5 to 30 parts by weight,
   in the case of glucoamylase: generally 0.001 to 5 parts by weight, preferably 0.1 to 1 parts by weight, and
(C): generally 0.1 to 5 parts by weight, preferably 1 to 5 parts by weight, more preferably 1 to 3 parts by weight.

Furthermore, the weight ratio of enzyme:additive other than enzyme in the enzyme preparation of the present invention may be, for example, 5 to 10:95 to 90, or the like.

In the case of a kit that is prepared separately and used together before use, the amount of enzyme contained in each preparation can be appropriately adjusted to achieve the above-mentioned activity.

The preparation of the present invention may be added at any step in the production process of the cheese analog, as described later. For example, the preparation of the present invention is generally used to be added in an amount of 0.1 to 50% by weight, preferably 0.5 to 10% by weight, more preferably 1 to 5% by weight, relative to the total weight of the cheese analog.

If the necessary amount of enzyme is extremely small, the preparation of the present invention may be prepared as an enzyme solution of a measurable concentration, and the solution may be diluted and then added.

The preparation of the present invention may further contain, in addition to the above-mentioned enzymes, excipients such as dextrin, starch, modified starch, and reduced maltose; seasonings such as meat extract; proteins such as plant protein, gluten, egg white, gelatin, and casein; protein hydrolysates, partially hydrolyzed proteins, emulsifiers, chelating agents such as citrates and polyphosphates; reducing agents such as glutathione and cysteine; alginic acid, lye water, fats and oils, pigments, acidulants, flavorings, and other food additives.

The preparation of the present invention may take the form of, for example, liquid, paste, granule, or powder.

### 2. Production method of cheese analog

The present invention also includes a method for producing a cheese analog, comprising (1) a step of reacting a mixture comprising a protein, starch, and fat or oil with (A) protease, (B) a carbohydrate-modifying enzyme, and (C) transglutaminase (hereinafter sometimes abbreviated as "the production method of the present invention").

In the production method of the present invention, (1) a step of reacting the enzymes (A) to (C) on the mixture is not particularly limited as long as the enzymes can be in contact with or coexist with the mixture. For example, the enzymes may be brought into direct contact with the mixture, or an enzyme solution may be prepared using the enzymes and water and then brought into contact with the mixture. Furthermore, the enzymes (A) to (C) may be brought into contact with the mixture simultaneously or with a time difference.

The step of reacting also includes a step of mixing the above-mentioned mixture with an enzyme and heating the mixture to a temperature suitable for reacting the enzyme. The heating step may also be a step of heating while stirring. More specifically, the step of reacting is a step of heating and mixing (emulsifying) while stirring fat or oil, starch, protein, optionally added additives (e.g., seasonings, various amino acids, excipients, flavors, colorings, thickening polysaccharides), and enzymes (A) to (C), and simultaneously allowing the enzymes (A) to (C) to react with the fat or oil, starch, and protein in the mixture (enzyme reaction step).

The heating temperature is generally 30 to 90°C, preferably 40 to 80°C, more preferably 50 to 70°C.

The heating time is generally 0.1 to 60 min, preferably 1 to 30 min, more preferably 3 to 10 min.

The pH at the time of contact with each enzyme is not particularly limited, and is generally pH 4 to 10.

In step (1) of the production method of the present invention, the amount of each enzyme to be added per gram of the mixture (cheese analog) is as follows:
(A): endo-type: generally 0.001 to 100000U, preferably 0.01 to 10000U, more preferably 0.1 to 1000U,
   exo-type: generally 0.0001 to 10000U, preferably 0.001 to 1000U, more preferably 0.01 to 100U,
   endo/exoprotease: generally 0.001 to 100000U, preferably 0.01 to 10000U, more preferably 0.1 to 1000U; when endo/exoprotease is used, the ratio of endoprotease activity and exoprotease activity in the endo/exoprotease (endoprotease
   activity:exoprotease activity) is generally 1U:0.000000001 to 10000000U, preferably 1U:0.0000001 to 100000U, more preferably 1U:0.00001 to 1000U (hereinafter the same),
(B): generally 0.0001 to 100000U, preferably 0.001 to 10000U, more preferably 0.01 to 1000U, and
(C): generally 0.0001 to 1000U, preferably 0.001 to 100U, more preferably 0.01 to 10U.

More specifically, in step (1) of the production method of the present invention, the amount of each enzyme to be added per gram of the mixture (cheese analog) is as follows:
(A): endo-type: generally 0.001 to 100000U, preferably 0.01 to 10000U, further preferably 0.1 to 5000U, more preferably 0.1 to 1000U,
   exo-type: generally 0.0001 to 10000U, preferably 0.001 to 1000U, more preferably 0.01 to 100U,
   endo/exoprotease: generally 0.001 to 100000U, preferably 0.01 to 10000U, further preferably 0.1 to 5000U, more preferably 0.1 to 1000U; when endo/exoprotease is used, the ratio of endoprotease activity and exoprotease activity in the endo/exoprotease (endoprotease activity:exoprotease activity) is generally 1U:0.000000001 to 10000000U, preferably 1U:0.0000001 to 100000U, more preferably 1U:0.00001 to 1000U (hereinafter the same),
(B): in the case of α-glucosidase: generally 0.0001 to 10000U, preferably 0.001 to 1000U, more preferably 0.01 to 100U,
   in the case of glucoamylase: generally 0.001 to 100000U, preferably 0.01 to 10000U, more preferably 0.1 to 1000U, and
(C): generally 0.0001 to 1000U, preferably 0.001 to 100U, more preferably 0.01 to 10U.

More specifically, in step (1) of the production method of the present invention, the amount of each enzyme to be added per gram of protein in the mixture is as follows:
(A): endo-type: generally 0.01 to 1000000U, preferably 0.1 to 100000U, more preferably 1 to 10000U,
   exo-type: generally 0.001 to 100000U, preferably 0.01 to 10000U, more preferably 0.1 to 1000U,
   endo/exo-type: generally 0.01 to 1000000U, preferably 0.1 to 100000U, more preferably 1 to 10000U,
(B): generally 0.001 to 1000000U, preferably 0.01 to 100000U, more preferably 0.1 to 10000U, and
(C): generally 0.001 to 10000U, preferably 0.01 to 1000U, more preferably 0.1 to 100U.

More particularly, in step (1) of the production method of the present invention, the amount of each enzyme to be added per gram of protein in the mixture is as follows:
(A): endo-type: generally 0.01 to 1000000U, preferably 0.1 to 100000U, further preferably 1 to 50000U, more preferably 1 to 10000U,
   exo-type: generally 0.001 to 100000U, preferably 0.01 to 100000, more preferably 0.1 to 1000U,
   endo/exo-type: generally 0.01 to 1000000U, preferably 0.1 to 100000U, further preferably 1 to 50000U, more preferably 1 to 10000U,
(B): in the case of α-glucosidase: generally 0.001 to 100000U, preferably 0.01 to 10000U, more preferably 0.1 to 1000U,
   in the case of glucoamylase: generally 0.01 to 1000000U, preferably 0.1 to 100000U, more preferably 1 to 10000U,
(C): generally 0.001 to 10000U, preferably 0.01 to 1000U, more preferably 0.1 to 100U.

Similarly in step (1) of the production method of the present invention, the amount of each enzyme to be added per gram of starch in the mixture is as follows:
(A): endo-type: generally 0.005 to 500000U, preferably 0.05 to 50000U, more preferably 0.5 to 5000U,
   exo-type: generally 0.0005 to 50000U, preferably 0.005 to 5000U, more preferably 0.05 to 500U,
   endo/exo-type: generally 0.005 to 500000U, preferably 0.05 to 50000U, more preferably 0.5 to 5000U,
(B): generally 0.0005 to 500000U, preferably 0.005 to 50000U, more preferably 0.05 to 5000U, and
(C): generally 0.0005 to 5000U, preferably 0.005 to 500U, more preferably 0.05 to 50U.

More specifically, in step (1) of the production method of the present invention, the amount of each enzyme to be added per gram of starch in the mixture is as follows:
(A): endo-type: generally 0.005 to 500000U, preferably 0.05 to 50000U, further preferably 0.5 to 25000U, more preferably 0.5 to 5000U,
   exo-type: generally 0.0005 to 50000U, preferably 0.005 to 5000U, more preferably 0.05 to 500U,
   endo/exo-type: generally 0.005 to 500000U, preferably 0.005 to 50000U, further preferably 0.5 to 25000U, more preferably 0.05 to 5000U,
(B): in the case of α-glucosidase: generally 0.0005 to 50000U, preferably 0.005 to 5000U, more preferably 0.05 to 500U,
   in the case of glucoamylase: generally 0.005 to 500000U, preferably 0.05 to 50000U, more preferably 0.5 to 5000U, and
(C): generally 0.0005 to 5000U, preferably 0.005 to 500U, more preferably 0.05 to 50U.

In the production method of the present invention, when (A) endoprotease and (B) are used, the ratio of the amounts of endoprotease and (B) to be added (endoprotease: (B) α-glucosidase) is, for example, 1U:0.000000001 to 10000000U, preferably 1U:0.0000001 to 100000U, more preferably 1U:0.00001 to 1000U.

In the production method of the present invention, when (A) endoprotease and (B) glucoamylase are used, the ratio of the amounts of endoprotease and (B) to be added (endoprotease: (B)) is, for example, 1U:0.00000001 to 100000000U, preferably 1U:0.000001 to 1000000U, more preferably 1U:0.0001 to 10000U.

In the production method of the present invention, when (A) exoprotease and (B) α-glucosidase are used, the ratio of the amounts of exoprotease and (B) to be added (exoprotease: (B)) is generally 1U:0.00000001 to 100000000U, preferably 1U:0.000001 to 1000000U, more preferably 1U:0.0001 to 10000U.

In the production method of the present invention, when (A) exoprotease and (B) glucoamylase are used, the ratio of the amounts of exoprotease and (B) to be added (exoprotease: (B)) is generally 1U:0.000000001 to 10000000U, preferably 1U:0.0000001 to 100000U, more preferably 1U:0.00001 to 1000U.

In the production method of the present invention, when (A) endo/exoprotease and (B) α-glucosidase are used, the ratio of the amounts of endo/exoprotease and (B) to be added (endo/exoprotease: (B)) is, for example, 1U:0.000000001 to 10000000U, preferably 1U:0.0000001 to 100000U, more preferably 1U:0.00001 to 1000U.

In the production method of the present invention, when (A) endo/exoprotease and (B) glucoamylase are used, the ratio of the amounts of endo/exoprotease and (B) to be added (endo/exoprotease: (B)) is, for example, 1U:0.00000001 to 100000000U, preferably 1U:0.000001 to 1000000U, more preferably 1U:0.0001 to 10000U.

In the production method of the present invention, when (A) endoprotease and (C) are used, the ratio of the amounts of endoprotease and (C) to be added (endoprotease: (C)) is, for example, 1U:0.000000001 to 1000000U, preferably 1U:0.0000001 to 10000U, more preferably 1U:0.00001 to 100U.

In the production method of the present invention, when (A) exoprotease and (C) are used, the ratio of the amounts of exoprotease and (C) to be added (exoprotease: (C)) is, for example, 1U:0.00000001 to 10000000U, preferably 1U:0.000001 to 100000U, more preferably 1U:0.0001 to 1000U.

In the production method of the present invention, when (A) endo/exoprotease and (C) are used, the ratio of the amounts of (A) endo/exoprotease protease and (C) to be added ((A) endo/exoprotease: (C)) is, for example, 1U:0.000000001 to 1000000U, preferably 1U:0.0000001 to 10000U, more preferably 1U:0.00001 to 100U.

In the production method of the present invention, when (B) α-glucosidase and (C) are used, the ratio of the amounts of (B) and (C) to be added ((B): (C)) is generally 1U:0.00000001 to 10000000U, preferably 1U:0.000001 to 100000U, more preferably 1U:0.0001 to 1000U.

In the production method of the present invention, when (B) glucoamylase and (C) are used, the ratio of the amounts of (B) and (C) to be added ((B):(C)) is generally 1U:0.000000001 to 1000000U, preferably 1U:0.0000001 to 10000U, more preferably 1U:0.00001 to 100U.

The production method of the present invention is characterized by including a step of reacting the enzymes of (A) to (C) on the above-mentioned mixture, which is a cheese analog raw material. Other steps that can be applied are conventional ones such as the following:
(2) a step of heating the mixture obtained in step (1),
(3) a step of cooling the heated mixture, and
(4) a step of cutting the cooled mixture.

In addition to the above, a step of adding other seasoning liquids or additives, a step of mixing with seasoning liquids, a step of stirring them, and the like may be included as appropriate.

For example, the mixing step and stirring step can be performed by methods known in the food production field. For example, mixing can be performed using a mixer used in the production of cheeses, such as a food processor, cooker-type emulsifier, kettle-type emulsifier, vertical high-speed shear emulsifier, or scraped surface heat exchanger.

In the production method of the present invention, the above-mentioned step (2) more specifically includes a step of further heating the mixture obtained in step (1) while stirring at a temperature at which starch gelatinizes to obtain a mixture containing the gelatinized starch, and a step of sterilizing.

The heating temperature in the above-mentioned step (2) is not particularly limited as long as enzymes are deactivated and further sterilized, and is generally 50 to 120°C, preferably 60 to 120°C, more preferably 70 to 120°C.

The heating time is generally 0.1 to 60 min, preferably 1 to 60 min, more preferably 5 to 60 min.

In the production method of the present invention, the above-mentioned step (3) is more specifically a step of pouring the mixture obtained in the above-mentioned step (2) into a mold and cooling same to obtain a cheese analog.

The cooling method in the above-mentioned step (3) is not particularly limited, and the heated product obtained in the above-mentioned step (2) is generally cooled to -20 to 20°C, preferably -10 to 10°C, more preferably 0 to 10°C. The heated product obtained in the above-mentioned step (2) may be filled in a desired container and cooled.

In the production method of the present invention, the above-mentioned step (4) more specifically includes a step of cutting the cheese analog obtained in step (3) into a block shape, and a step of cutting it into the form of sliced cheese or shredded cheese. For example, these steps allow for the production of shredded cheese by shredding into a predetermined size. The specific shred size can be adjusted as appropriate and is not particularly limited. It is, for example, 4.5 to 10 mm x 30 mm, and for fine shredding, 1.0 to 2.0 mm x 60 mm.

In the production method of the present invention, the pH of the mixture containing fat or oil, starch, and protein on which (A) to (C) are reacted is, for example, pH 3 to 6.

In the production method of the present invention, the mixture containing fat or oil, starch, and protein on which (A) to (C) are reacted contains water.

The amount of water to be used is, for example, 5 to 80% by weight, preferably 15 to 70% by weight, more preferably 25 to 60% by weight, further preferably 35 to 50% by weight, relative to the cheese analog.

In the production method of the present invention, the cheese analog may contain additives generally used in the food industry and other than the above-mentioned components.

Examples of additives include flavors (e.g., cheddar flavoring (powder, liquid), parmesan flavor (powder, liquid), camembert flavor (powder, liquid), cream cheese flavor (powder, liquid)), seasonings (e.g., salt, yeast extract, lactic acid), colorants, excipients (dextrin, lactose), various amino acids, thickening polysaccharides (e.g., gum arabic, xanthan gum, tamarind seed gum, guar gum, locust bean gum, carrageenan, agar), proteases, carbohydrate-modifying enzymes, and enzymes other than TG.

The amount of additive to be used is, for example, 1 to 30% by weight relative to the cheese analog.

The definitions and preferred ranges of (A) to (C) and cheese analogs in the production method of the present invention are the same as those described for the preparation of the present invention.

The cheese analog produced by the production method of the present invention can be used as is or as a food in combination with other food ingredients or general foods.

In the present specification, "food" is a broad concept encompassing anything that can be ingested orally (excluding pharmaceutical products), and includes not only so-called, "foods" but also beverages, health supplements, functional foods (e.g., Foods for Specified Health Uses, Foods with Function Claims, Foods with Nutrient Function Claims), supplements, and the like.

Examples of foods using the cheese analog produced by the production method of the present invention include foods that are consumed after heating, such as pizza, gratin, doria, and lasagna.

The present invention also includes a method for increasing the hardness of a cheese analog, comprising a step of reacting a mixture comprising a protein, starch, and fat or oil with (A) a protease, (B) a carbohydrate modifying enzyme, and (C) a transglutaminase (hereinafter also abbreviated as the hardness-increasing method of the present invention).

In the hardness-increasing method of the present invention, when a mixture containing fat or oil, starch, and protein is reacted with (A) protease and (B) carbohydrate-modifying enzyme, the hardness at room temperature becomes lower than the hardness suitable for cutting. Therefore, the hardness-increasing method of the present invention is characterized by further reacting the mixture with (C) transglutaminase in order to increase the hardness and improve the efficiency of production such as cutting.

Increasing hardness means raising or adjusting the hardness to a level suitable for cutting. A hardness suitable for cutting refers to the general hardness of so-called hard cheese, which is used after shredding. The hardness suitable for cutting refers to a shredding rate of 90% or more.

The definitions and preferred ranges of (A) to (C) and cheese analogs in the the hardness-increasing method of the present invention are the same as those described for the production method of the present invention.

### [Example]

The present invention is described in more detail below based on Examples and Experimental Examples, but the present invention is not limited to these.

### [Experimental Example 1]

### Prototype Procedure

The raw materials, weighed according to the mixing ratios shown in Table 1, were emulsified by heating them at 70°C for 5 min with stirring (enzyme reaction step) using a heating mixer (Thermomix TM21, manufactured by Vorwek) according to the flowchart in Fig. 1. Subsequently, they were heated at 90°C for 8 min with stirring (enzyme inactivation step). The obtained mixture was filled in a mold and cooled in a refrigerator (5°C) for 48 hr to obtain a cheese analog.

In addition, to examine the properties of the cheese analog, the cheese was cut into circular shape.

**[Table 1]**

| raw materials | mixing ratio (wt%) | | | |
|---|---|---|---|---|
| | 1A | 2A | 3A | 4A |
| waxy corn starch | 10.17 | 10.17 | 10.17 | 10.17 |
| tapioca starch | 10.30 | 10.30 | 10.30 | 10.30 |
| gum arabic | 1.83 | 1.83 | 1.83 | 1. 83 |
| tamarind seed gum | 0.30 | 0.30 | 0.30 | 0.30 |
| yeast extract | 0.95 | 0.95 | 0.95 | 0.95 |
| coconut oil | 20.00 | 20.00 | 20.00 | 20.00 |
| water | 40.50 | 40.50 | 40.50 | 40.50 |
| green pea protein (protein 84%) | 5.95 | 5.95 | 5.95 | 5.95 |
| broad bean protein (protein 60%) | 8.30 | 8.30 | 8.30 | 8.30 |
| salt | 1.40 | 1.40 | 1.40 | 1.40 |
| lactic acid | 0.30 | 0.30 | 0.30 | 0.30 |
| total | 100.00 | 100.00 | 100.00 | 100.00 |
| AG (α-glucosidase) | | 0.30 | 0.30 | 0.30 |
| PRO (protease) | | 0.20 | 0.20 | 0.20 |
| TG (transglutaminase) | | | 0.01 | 0.03 |

| | | | | |
|---|---|---|---|---|
| AG: α-glucosidase, manufactured by Amano Enzyme Inc., 120U/g. The amount of α-glucosidase in the cheese analogs of test plots 2A to 4A, when converted to enzyme activity per gram of cheese analog, was 0.36 U. PRO: ProteAX (product name), manufactured by Amano Enzyme Inc., 218,000U/g (endo-type activity), 1,400 U/g (exo-type activity) (endo/exoprotease). The amount of protease in the cheese analogs of test plots 2A to 4A, when converted to enzyme activity per gram of cheese analog, was 436 U for endo-type activity and 2.8 U for exo-type activity. TG: transglutaminase, 1300U/g. The amount of transglutaminase in the cheese analogs of test plot 3A, when converted to enzyme activity per gram of cheese analog, was 0.13 U. | | | | |

### Measurement method

### (Evaluation of meltability of cheese analog when heated)

The meltability of the cheese analog when heated was evaluated by the method shown in Fig. 2.

A cheese analog (weight: 2 g, size: diameter 2 cm, thickness: 2 mm) was cut out, placed on a circular sheet, and heated in an oven at 180°C for 10 min.

After completion of heating, the distance of spreading of the melted cheese analog was measured at four points on the circular sheet, and the average value was calculated.

The meltability was evaluated from the calculated value, according to the criteria shown below.
⊙: 3.51 mm or more
○: 2.76 to 3.50 mm
Δ: 2.01 to 2.75 mm
×: 2.00 mm or less

### (Evaluation of shreddability)

According to the method shown in Fig. 2, a cheese analog (weight: 70 g, size: 5 cm wide, 3 cm long, 3 cm high) was shredded at a laboratory temperature of 15°C using a shredder (Professional SaladShooter Electric Slicer, manufactured by Presto, 15 shredding holes x 2 rows).

The shredding rate was calculated from the amount of cheese analog remaining in the shredder and the amount of shredded cheese analog.

The shreddability was evaluated based on the calculated shredding rate and according to the criteria shown below.
⊙: 96.1% or more
○: 93.1% to 96.0%
Δ: 90.1% to 93.0%
×: 90.0% or less

### Effect of TG addition

The samples in Table 1 were prepared and evaluated by the method of Fig. 2 and according to the criteria described above. The results are shown in Table 2 and Fig. 3.

**[Table 2]**

| sample No. | | 1A | 2A | 3A | 4A |
|---|---|---|---|---|---|
| enzyme concentration (concentration at the time of eating) | AG (wt%) | 0.00% | 0.30% | 0.30% | 0.30% |
| | PRO (wt%) | 0.00% | 0.20% | 0.20% | 0.20% |
| | TG (wt%) | 0.00% | 0.00% | 0.01% | 0.03% |
| meltability when heated (quantitative) | meltability (mm) | 1.92 | 4.08 | 3.75 | 3.67 |
| meltability when heated (qualitative) | meltability | × | ⊙ | ⊙ | ⊙ |
| shreddability (quantitative) | shredding rate | 98.5% | 93.0% | 94.8% | 95.8% |
| shreddability (qualitative) | shreddability | ⊙ | Δ | ○ | ○ |

When an enzyme was not added, cheese analogs did not melt and spread much when heated. However, the addition of AG and PRO increased melting and spreading thereof (improved meltability). The cheese analogs added with AG and PRO showed a decrease by 5% or more in the shredding rate compared to when no enzymes were added, and a large amount of cheese analog was left in the shredder, highly possibly leading to decreased productivity. It was confirmed that reacting TG here improved the shredding rate while maintaining meltability.

### [Experimental Example 2]

### Study of TG concentration

The samples in Table 3 were prepared in the same manner as in Experimental Example 1 and evaluated by the method of Fig. 2 and according to the criteria in Experimental Example 1. The results are shown in Table 4 and Fig. 4.

**[Table 3]**

| raw materials | mixing ratio (wt%) | | | | | |
|---|---|---|---|---|---|---|
| | 1B | 2B | 3B | 4B | 5B | 6B |
| waxy corn starch | 10.17 | 10.17 | 10.17 | 10.17 | 10.17 | 10.17 |
| tapioca starch | 10.30 | 10.30 | 10.30 | 10.30 | 10.30 | 10.30 |
| gum arabic | 1.83 | 1.83 | 1.83 | 1.83 | 1.83 | 1.83 |
| tamarind seed gum | 0.30 | 0.30 | 0.30 | 0.30 | 0.30 | 0.30 |
| yeast extract | 0.95 | 0.95 | 0.95 | 0.95 | 0.95 | 0.95 |
| coconut oil | 20.00 | 20.00 | 20.00 | 20.00 | 20.00 | 20.00 |
| water | 40.50 | 40.50 | 40.50 | 40.50 | 40.50 | 40.50 |
| green pea protein (protein 84%) | 5.95 | 5.95 | 5.95 | 5.95 | 5.95 | 5.95 |
| broad bean protein (protein 60%) | 8.30 | 8.30 | 8.30 | 8.30 | 8.30 | 8.30 |
| salt | 1.40 | 1.40 | 1.40 | 1.40 | 1.40 | 1.40 |
| lactic acid | 0.30 | 0.30 | 0.30 | 0.30 | 0.30 | 0.30 |
| total | 100.00 | 100.00 | 100.00 | 100.00 | 100.00 | 100.00 |
| AG | 0.30 | 0.30 | 0.30 | 0.30 | 0.30 | 0.30 |
| PRO | 0.20 | 0.20 | 0.20 | 0.20 | 0.20 | 0.20 |
| TG | | 0.01 | 0.02 | 0.03 | 0.04 | 0.05 |

**[Table 4]**

| sample No. | | 1B | 2B | 3B | 4B | 5B | 6B |
|---|---|---|---|---|---|---|---|
| enzyme concentration (concentration at the time of eating) | AG | 0.30% | 0.30% | 0.30% | 0.30% | 0.30% | 0.30% |
| | PRO | 0.20% | 0.20% | 0.20% | 0.20% | 0.20% | 0.20% |
| | TG | 0.00% | 0.01% | 0.02% | 0.03% | 0.04% | 0.05% |
| meltability when heated (quantitative) | meltability (mm) | 3.92 | 3.92 | 3.58 | 3.42 | 3.25 | 3.08 |
| meltability when heated (qualitative) | meltability | ⊙ | ⊙ | ⊙ | ○ | ○ | ○ |
| shreddability (quantitative) | shredding rate | 89.8% | 91.0% | 91.5% | 93.1% | 95.2% | 94.5% |
| shreddability (qualitative) | shreddability | × | Δ | Δ | ○ | ○ | ○ |

It was found that adding 0.01% or more of TG improved the shredding rate, and that adding 0.03% or more markedly improved the shredding rate. It was also found that even with addition of 0.05% TG, the meltability was higher compared to the enzyme-free cheese analog.

### [Experimental Example 3]

### Study of GA concentration

The samples in Table 5 were prepared in the same manner as in Experimental Example 1 except that AG was changed to glucoamylase (GA) and evaluated by the method of Fig. 2 and according to the criteria in Experimental Example 1. The results are shown in Table 6 and Fig. 5.

**[Table 5]**

| raw materials | mixing ratio (wt%) | | | |
|---|---|---|---|---|
| | 1C | 2C | 3C | 4C |
| waxy corn starch | 10.17 | 10.17 | 10.17 | 10.17 |
| tapioca starch | 10.30 | 10.30 | 10.30 | 10.30 |
| gum arabic | 1.83 | 1. 83 | 1.83 | 1.83 |
| tamarind seed gum | 0.30 | 0.30 | 0.30 | 0.30 |
| yeast extract | 0.95 | 0.95 | 0.95 | 0.95 |
| coconut oil | 20.00 | 20.00 | 20.00 | 20.00 |
| water | 40.50 | 40.50 | 40.50 | 40.50 |
| green pea protein | 5.95 | 5.95 | 5.95 | 5.95 |
| broad bean protein | 8.30 | 8.30 | 8.30 | 8.30 |
| salt | 1.40 | 1.40 | 1.40 | 1.40 |
| lactic acid | 0.30 | 0.30 | 0.30 | 0.30 |
| total | 100.00 | 100.00 | 100.00 | 100.00 |
| GA (glucoamylase) | 0.001 | 0.001 | 0.001 | 0.001 |
| PRO (protease) | 0.20 | 0.20 | 0.20 | 0.20 |
| TG (transglutaminase) | | 0.01 | 0.03 | 0.05 |

| | | | | |
|---|---|---|---|---|
| GA: Glucoamylase for sake brewing "Amano" SD (product name), manufactured by Amano Enzyme Inc., 250,000U/g. The amount of glucoamylase in the cheese analogs of test plots 1C to 4C, when converted to enzyme activity per gram of cheese analog, was 2.5 U. | | | | |

**[Table 6]**

| sample No. | | 1C | 2C | 3C | 4C |
|---|---|---|---|---|---|
| enzyme concentration (concentration at the time of eating) | GA (wt%) | 0.001% | 0.001% | 0.001% | 0.001% |
| | PRO (wt%) | 0.20% | 0.20% | 0.20% | 0.20% |
| | TG (wt%) | 0.00% | 0.01% | 0.03% | 0.05% |
| meltability when heated (quantitative) | meltability (mm) | 4.08 | 4.08 | 4.17 | 4.08 |
| meltability when heated (qualitative) | meltability | ⊙ | ⊙ | ⊙ | ⊙ |
| shreddability (quantitative) | shredding rate | 89.2% | 93.7% | 93.4% | 93.6% |
| shreddability (qualitative) | shreddability | × | ○ | ○ | ○ |

It was found that, even when GA and PRO were used to improve meltability, addition of 0.01% or more of TG improved the shredding rate.

### [Experimental Example 4]

### Study of lentil protein

The samples in Table 7 were prepared in the same manner as in Experimental Example 1 except that protein was changed to lentil protein and evaluated by the method of Fig. 2 and according to the criteria in Experimental Example 1. The results are shown in Table 8 and Fig. 6.

**[Table 7]**

| raw materials | mixing ratio (wt%) | | |
|---|---|---|---|
| | 1D | 2D | 3D |
| waxy corn starch | 10.17 | 10.17 | 10.17 |
| tapioca starch | 10.30 | 10.30 | 10.30 |
| gum arabic | 1.83 | 1.83 | 1.83 |
| tamarind seed gum | 0.30 | 0.30 | 0.30 |
| yeast extract | 0.95 | 0.95 | 0.95 |
| coconut oil | 20.00 | 20.00 | 20.00 |
| water | 40.50 | 40.50 | 40.50 |
| lentil protein (protein 70%) | 14.25 | 14.25 | 14.25 |
| salt | 1.40 | 1.40 | 1.40 |
| lactic acid | 0.30 | 0.30 | 0.30 |
| total | 100.00 | 100.00 | 100.00 |
| GA (glucoamylase) | | 0.001 | 0.001 |
| PRO (protease) | | 1.00 | 1.00 |
| TG (transglutaminase) | | | 0.03 |

| | | | |
|---|---|---|---|
| GA: Glucoamylase for sake brewing "Amano" SD (product name), manufactured by Amano Enzyme Inc., 250,000U/g. The amount of glucoamylase in the cheese analogs of test plots 2D and 3D, when converted to enzyme activity per gram of cheese analog, was 2.5 U. PRO: ProteAX (product name), manufactured by Amano Enzyme Inc., 218,000U/g (endo-type activity), 1,400U/g (exo-type activity) (endo/exoprotease). The amount of protease in the cheese analogs of test plots 2D and 3D, when converted to enzyme activity per gram of cheese analog, was 2180 U for endo-type activity and 14 U for exo-type activity. TG: transglutaminase, 1300U/g. The amount of transglutaminase in the cheese analog of test plot 3D, when converted to enzyme activity per gram of cheese analog, was 0.13 U. | | | |

**[Table 8]**

| sample No. | | 1D | 2D | 3D |
|---|---|---|---|---|
| enzyme concentration (concentration at the time of eating) | GA (wt%) | 0.000% | 0.001% | 0.001% |
| | PRO (wt%) | 0.00% | 1.00% | 1.00% |
| | TG (wt%) | 0.00% | 0.00% | 0.03% |
| meltability when heated (quantitative) | meltability (mm) | 2.00 | 5.00 | 5.00 |
| meltability when heated (qualitative) | meltability | × | ⊙ | ⊙ |
| shreddability (quantitative) | shredding rate | 99.6% | 50.7% | 94.0% |
| shreddability (qualitative) | shreddability | ⊙ | × | ○ |

When no enzymes were added, even cheese analogs using lentil protein did not melt and spread much when heated (1D). However, when GA and PRO were added, the melting and spreading increased from 2 mm to 5 mm (2D) (improved meltability). Furthermore, it was confirmed that even when lentil protein was used, the shredding rate improved dramatically from 50.7% to 94.0% while maintaining meltability, by reacting TG (3D).
(materials used)
waxy corn starch: waxy corn starch MD (NIHON SHOKUHIN KAKO) tapioca starch: NOVATION3300 (Ingredion)
gum arabic: Superstab AA (Nexira)
tamarind seed gum: Glyloid 2A (Sumitomo Pharma)
coconut oil: Organic Premium Coconut Oil Cocowell (Cocowell) green pea protein (Pea Protein): Nutralys F85M (ROQUETTE) broad bean protein (Fava Protein): VITESSENCE Prista P360
(Ingredion)
salt: Nakuru M (Naikai Salt Industries Co., Ltd.)
lactic acid: Lactic Acid Powder (Mezzoni Foods)
PRO: protease: Proteax (Amano Enzyme Inc.)
AG (α-Glucosidase): α-glucosidase "Amano", Amano Enzyme Inc.
GA: glucoamylase for sake brewing "Amano" SD, Amano Enzyme Inc. lentil protein: Organic lentil protein (GUZEN)

### [Industrial Applicability]

According to the present invention, shredded cheese, sliced cheese, and the like with improved meltability and/or stretchability when heated can be efficiently produced by reacting a mixture containing fat or oil, starch, and protein with (A) protease, (B) carbohydrate modifying enzyme, and (C) transglutaminase.

This application is based on a patent application No. 2023-194015 filed in Japan, the contents of which are encompassed in full herein.

## Claims

1. An enzyme preparation for a cheese analog, comprising (A) a protease, (B) a carbohydrate-modifying enzyme, and (C) a transglutaminase.

2. The preparation according to claim 1, wherein (B) is α-glucosidase, glucoamylase, or a mixture thereof.

3. The preparation according to claim 1, wherein (B) is glucoamylase.

4. The preparation according to claim 1, wherein the cheese analog comprises a protein, starch, and fat or oil.

5. The preparation according to claim 1, wherein the cheese analog is a finely-cut cheese analog.

6. The preparation according to claim 5, wherein the finely-cut cheese analog is a shredded cheese analog.

7. The preparation according to claim 1, comprising 100 to 1000000 U of (A), 0.1 to 100000 U of (B), and 0.1 to 200 U of (C), per 1 g of the enzyme preparation.

8. The preparation according to claim 7, which is added in an amount of 0.1 to 50% by weight relative to the total weight of the cheese analog.

9. A method for producing a cheese analog, comprising (1) a step of reacting a mixture comprising a protein, starch, and fat or oil with (A) protease, (B) a carbohydrate-modifying enzyme, and (C) transglutaminase.

10. The production method according to claim 9, wherein (B) is α-glucosidase, glucoamylase, or a mixture thereof.

11. The production method according to claim 9, wherein (B) is glucoamylase.

12. The production method according to claim 9, wherein the protein is a plant-derived protein.

13. The production method according to claim 9, further comprising
(2) a step of heating the mixture obtained in step (1),
(3) a step of cooling the heated mixture, and
(4) a step of cutting the cooled mixture.

14. The production method according to claim 13, wherein the cheese analog is a finely-cut cheese analog.

15. The production method according to claim 14, wherein the finely-cut cheese analog is a shredded cheese analog.

16. The production method according to claim 9, wherein step (1) is a step of reacting 0.001 to 100000 U of (A), 0.0001 to 100000 U of (B), and 0.0001 to 1000 U of (C), per 1 g of the mixture.

17. A method for increasing the hardness of a cheese analog, comprising a step of reacting a mixture comprising a protein, starch, and fat or oil with (A) a protease, (B) a carbohydrate modifying enzyme, and (C) a transglutaminase.
